(19) Europäisches Patentamt
European Patent Office
Office européen des brevets

(11) EP 2 610 242 A1

(12) EUROPEAN PATENT APPLICATION

(43) Date of publication:
**03.07.2013 Bulletin 2013/27**

(21) Application number: **13155198.8**

(22) Date of filing: **09.07.2006**

(51) Int Cl.:
*C07C 219/14* (2006.01) *C07C 235/60* (2006.01)
*C07C 327/30* (2006.01) *A61K 31/235* (2006.01)
*A61P 29/00* (2006.01)

(84) Designated Contracting States:
**AT BE BG CH CY CZ DE DK EE ES FI FR GB GR HU IE IS IT LI LT LU LV MC NL PL PT RO SE SI SK TR**

(62) Document number(s) of the earlier application(s) in accordance with Art. 76 EPC:
**06780025.0 / 2 038 251**

(71) Applicants:
- **Techfields Biochem Co. Ltd**
**Shanghai**
**200444 (CN)**
- **Chongxi Yu**
**Plainfield, Illinois 60585 (US)**

(72) Inventors:
- **Yu, Chongxi**
**Plainfield, IL 60585 (US)**
- **Xu, Lina**
**200444 Shanghai (CN)**

(74) Representative: **Viering, Jentschura & Partner**
**Kennedydamm 55 / Roßstrasse**
**40476 Düsseldorf (DE)**

Remarks:
•This application was filed on 14-02-2013 as a divisional application to the application mentioned under INID code 62.
•Claims filed after the date of receipt of the divisional application (Rule 68(4) EPC).

# (54) Positively charged water-soluble prodrugs of aspirin

(57) The novel positively charged prodrugs of acetylsalicylic acid and its analogues in the general formula (1) 'Structure 1' were designed and synthesized. The compounds of the general formula(1) 'Structure 1' indicated above can be prepared from functional derivatives of ASA or its analogues,(for example acid halides or mixed anhydrides), by reaction with suitable alcohols, thiols, or amines. The positively charged amino groups of these pro-drugs not only largely increases the solubility of the drugs, but also bonds to the negative charge on the phosphate head group of membranes and push the pro-drug into the cytosol. The experiment results suggest that the pro-drug, diethylaminoethyl acetylsalicylate. AcOH, diffuses through human skin -400 times faster than acetylsalicylic acid itself and ~100 times faster than ethyl acetylsalicylate. In plasma, 80% of these pro-drugs can change back to the drug in a few minutes. The prodrugs can be used medicinally in treating any aspirin-treatable conditions in humans or animals and be administered not only orally, but also transdermally for any kind of medical treatments and avoid most of the side effects of aspirin, most notably GI disturbances such as dyspepsia, gastroduodenal bleeding, gastric ulcerations, and gastritis. Controlled transdermal administration systems of the prodrug enables the aspirin to reach constantly optimal therapeutic blood levels to increase effectiveness and reduce the side effects of aspirin.

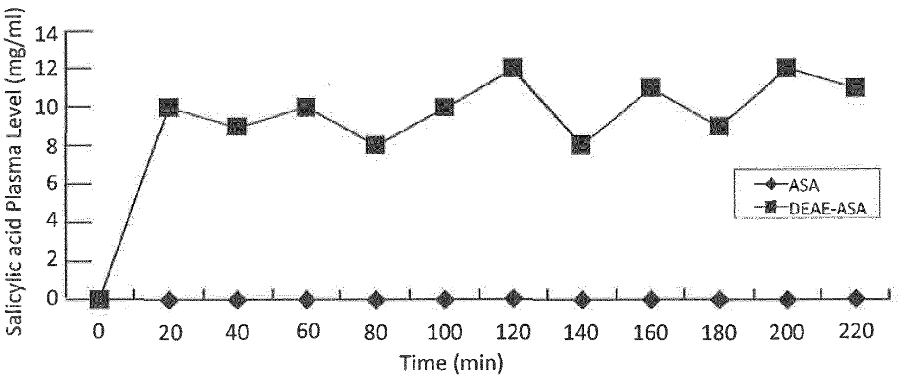


Fig. 2



Printed by Jouve, 75001 PARIS (FR)

## Description

## Technical Field

The present invention relates to the preparations of positively charged and watersoluble pro-drugs of aspirin or its analogues and their medicinal use in treating any aspirin-treatable conditions in humans or animals. Particularly, the present invention is to overcome the side effects that are associated with the use of salicylates. These pro-drugs can be administered orally or transdermally.

## Background Art

Acetylsalicylic acid (aspirin) was synthesized in 1853 and first used medicinally in 1899. Since then, numerous derivatives of salicylic acid have been synthesized and evaluated pharmacologically, yet relatively few derivatives have achieved therapeutic utility. Aspirin possesses antipyretic, analgesic, and anti-inflammatory properties. Because salicylates promote the excretion of uric acid, they are useful in the treatment of gouty arthritis. Aspirin also inhibits the platelet aggregation that potentially contributes to heart attacks and stroke [C. H. Hennekens, et al., N. Engl. J. Med., 321, 129(1989); T.A.Gossel, U.S. Pharmacist, February, 1988, p. 34.] and it may be protective against colon cancer as also [M.J.Thun, et al., N. Engl. J. Med., 325, 1393(1991)]. Thus, the therapeutic utility of aspirin is valuable and continues to increase. "PDR Generics" (PDR Generics, 1996, second edition, Medical Economics, Montvale, New Jersey, pg 243) has listed many medical uses of aspirin. Aspirin is also used in the long-term palliative treatment of mild to moderate pain and inflammation of arthritic and other inflammatory conditions. It is used alone or as an adjunct in the treatment of Kawasaki syndrome, thromboembolism after surgery, and unstable angina. It is also used to decrease stool volume and increase weight in acute childhood gastroenteritis, as an adjunct in preventing aortocoronary-artery-bypass graft occlusion, and used to prevent thromboembolic complications in chronic arterial fibrillation. It is used as an adjunct in carotid endarterectomy to reduce platelet aggregatioartrial n, prescribed to reduce the development of cataracts, used to prevent the recurrence of stenosis after coronary angioplasty, and used to improve cognitive performance and cerebral blood flow in patients with multi-infract dementia. It is used as an adjunct to lower plasma glucose in diabetes mellitus and diabetes-induced complications, including diabetic retinopathy, necrobiotic ulcers, and diabetic proteinuria, and to decrease total and cardiovascular mortality. It is prescribed to reduce the incidence of hemodialysis shunt thrombosis, to decrease the deterioration of renal function and the occurrence of end-stage renal disease in patients with type-1 membranoproliferative glomerulonephritis, and to slow progression of peripheral occlussive arterial disease. It is used in the prevention of colon cancer, rectal cancer, and arterial embolic complications in patients' prosthetic heart valves. It is also prescribed to lower the incidence of pregnancy-induced hypertension and preeclamptic toxemia in high-risk women.

Unfortunately, a number of side effects are associated with the use of salicylates, most notably GI disturbances such as dyspepsia, gastroduodenal bleeding, gastric ulcerations, and gastritis. Despite the vast effort expended in the search to find a "better" aspirin, a superior product has yet to be discovered. Aspirin has very low solubility in water and may stay in the GI tract for a long time, thus causing gastric mucosal cell damage. Stable salts of O-acetylsalicylic with base amino acids were studied for their use as medicaments (Franckowiak, et al., U.S. Pat. No. 6,773,724), but as the pH of gastric juice is 1-3, the salts may be acidified to acetylsalicylic acid. Esters of alkyl- or aralkyl-substituted acetylsalicylic acid such as methyl-, ethyl, allyl-, or benzyl acetylsalicylate were synthesized and studied (Boghosian, et al., U.S. Pat. No. 4,244,948). Higher fatty acid derivatives of salicylic acid and salts thereof were synthesized for use in treating aspirin-treatable conditions (Guttag, U.S. Pat. No. 5,760,261). Esters of salsalate with guaiacol were synthesized and evaluated pharmacologically (Nicolini, U.S. Pat. No. 4,743,704).

Transdermal administration of aspirin has many advantages (Kissel, U.S. Pat. No.5,861,170). 1. Aspirin is directly introduced into the systemic circulation in its pharmacologically active form, thus avoiding metabolism in the gastrointestinal tract; 2. reduction of gastrointestinal side effects; 3. constant therapeutic effect with reduced doses of aspirin; 4. reduced risk of overdosage; 5. treatment of outpatients without the need for observation; and 6. improved patient compliance rate. Attempts have already be made to apply aspirin or its analogues to skin. Burton (Burton, U.S. Pat. No.4,012,508) employed aspirin in combination with corticosteroids for topical application in the case of dermatological indications. Sibalis (Sibalis, U.S. Pat. No.4,640,689) described that there is an increase in the penetration rate of aspirin by employing an electric current. It is very difficult, however, to deliver therapeutically effective plasma levels of aspirin into the host by these methods. Susan Milosovich, et. al. designed and prepared testosteronyl-4-dimethylaminobutyrate.HCl (TSBH), which has a lipophilic portion and a tertiary amine groups that exists in the protonated form at physiologic pH. They found that the pro-drug (TSBH) diffuses through human skin -60 times faster than does the drug (TS) itself [Susan Milosovich, et al., J. Pharm. Sci., 82, 227(1993)].

## Disclosure of Invention

### Technical Problem

Aspirin is a very old salicylate drug (more than 100 years old) and has demonstrated anti-inflammatory, analgesic, antipyretic, and antirheumatic activities, it is also used to inhibit platelet aggregation, thus reducing cardiac mortality, and the therapeutic utility of aspirin continues to increase. "PDR Generics " (PDR Generics, 1996, second edition, Medical Economics, Montvale, New Jersey, pg 243) has listed many medical uses of aspirin. Unfortunately, a number of side effects are associated with the use of salicylates, most notably GI disturbances such as dyspepsia, heartburn, vomiting, gastroduodenal bleeding, gastric ulcerations, and gastritis. Gastroduodenal bleeding induced by salicylates is generally painless but can lead to fecal blood loss and may cause a persistent iron deficiency anemia. In a crossover study, uncoated aspirin at a dosage of 325 mg tablets given three times a day caused an average fecal blood loss of 4.33 ml per day (PDR Generics, 1996, second edition, Medical Economics, Montvale, New Jersey, pg 242). Coated aspirin at the same dosage caused an average fecal blood loss of 1.5 ml per day. Aspirin and its esters have a very low solubility in water and may stay in the GI tract for a long time and can cause gastric mucosal cell damage. Aspirin and its esters are also very hydrophobic, and when they enter the cell membrane (hydrophobic layer), they will stay there as part of the membrane due to their similarity. Due to these reasons, the absorption rate of aspirin is very slow. It takes 2 hours for uncoated aspirin tablets to reach the peak salicylate level and much longer for coated aspirin tablets.

### Technical Solution

This invention relates to the preparation of novel positively charged pro-drugs of acetylsalicylic acid or its analogues and their use medicinally. These pro-drugs have the general formula(1) "Structure 1".

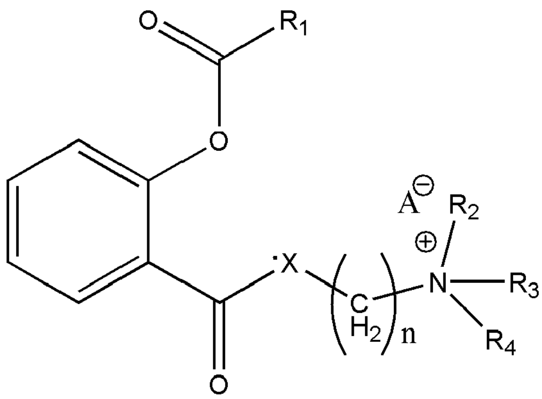

Structure 1

In which, $R_1$ represents $CH_3$, $C_2H_5$, $C_3H_7$, or other lower alkyl groups; $R_2$ represents H, one of any alkyl, alkyloxy, or alkenyl residues having 1 to 6 carbon atoms, or aryl residues; $R_3$ represents H, one of any alkyl, alkyloxy, or alkenyl residues having 1 to 6 carbon atoms, or aryl residues; $R_4$ represents H, one of any alkyl, alkyloxy, or alkenyl residues having 1 to 6 carbon atoms, or aryl residues; X represents O, S or NH; $A^-$ represents $Cl^-$, $Br^-$, F, $I^-$, $AcO^-$, acetylsalicylate, citrate, salicylate, or any negative ions; and n=0,1,2,3,4,5......

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs can not pass the hydrophobic layer of membrane and very hydrophobic drugs will stay in the hydrophobic layer as part of the membrane due to their similarities and cannot enter the cytosol on the inside efficiently.

The goal of this invention is to avoid the side effects of aspirin by increasing the solubility of acetylsalicylic acid in gastric juice and the penetration rate of aspirin through the membrane and skin barrier which will make it administrable transdermally (topical application). These novel pro-drugs of acetylsalicylic acid have two structural features in common: they have a lipophilic portion and a primary, secondary, or tertiary amine group that exists in the protonated form

(hydrophilic part) at physiologic pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs. The solubilities of diethylaminoethyl acetylsalicylate.AcOH and acetylsalicylic acid in pH 7 phosphate buffer were >300 mg and 0.01 mg/ml. In many instances, the slowest or rate-limiting step in the sequence is the dissolution of the drug. Aspirin has a very low solubility in gastric juice. It stays in the GI tract for a long time and may cause gastric mucosal cell damage. When these new pro-drugs are administered orally in a dosage form such as a tablet, capsule, solution, or suspension, they will dissolve in the gastric juice immediately. The positive charge on the amino groups of these pro-drugs will bond to the negative charge on the phosphate head group of membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. Due to the short stay in GI tract, the pro-drugs will not cause gastric mucosal cell damage.

The penetration rates of diethylaminoethyl acetylsalicylate.AcOH, ethyl acetylsalicylate, and acetylsalicylic acid through human skin were measured in vitro by using modified Franz cells, which were isolated from human skin tissue (360-400 μm thick) of the anterior and posterior thigh areas. The receiving fluid consisted of 10 ml of 2% bovine serum albumin in normal saline and was stirred at 600 rpm. The cumulative amounts of acetylsalicylic acid , ethyl acetylsalicylate, and diethylaminoethyl acetylsalicylate.AcOH penetrating the skin versus time were determined by a specific high-performance liquid chromatography method. The results using a donor consisting of either a 20% suspension of acetylsalicylic acid and ethyl acetylsalicylate or 20% solution of diethylaminoethyl acetylsalicylate.AcOH in 2mL of pH 7.4 phosphate buffer (0.2M) are shown in Figure 1. Apparent flux values of 0.25 mg, 1 mg, and 100 mg/cm$^2$/h were calculated for acetylsalicylic acid, ethyl acetylsalicylate (the no positive charged normal ester of acetylsalicylic acid), and diethylaminoethyl acetylsalicylate .AcOH. The results suggest that the pro-drug, diethylaminoethyl acetylsalicylate.AcOH, diffuses through human skin -400 times faster than does acetylsalicylic acid itself and ~100 times faster than does ethyl acetylsalicylate. The normal ester, ethyl acetylsalicylate and acetylsalicylic acid itself have very similar penetration rate (only 4 times difference). The results suggest that the positive charge on the di-alkyaminoethyl group has a very important role in the passage of the drug across the membrane and skin barrier. Other pro-drugs of the general "Structure 1" have very high penetration rates and are very close to that of diethylaminoethyl acetylsalicylate.AcOH.

The in vivo rates of penetration of acetylsalicylic acid and diethylaminoethyl acetylsalicylate.AcOH through the skin of intact hairless mice were compared. The donor consisted of either a 30% suspension of acetylsalicyclic acid or a 30% solution of diethylaminoethyl acetylsalicylate.AcOH in 1 mL of isopropanol applied to a 1 cm$^2$ area on the backs of the hairless mice. Plasma levels of salicylic acid (acetylsalicyclic acid has a very short half live, ~15 minutes in plasma, so the amount of it is difficult to be determined) were determined by a specific high-performance liquid chromatography method. The results (Figure 2) show that the peak levels of salicylic acid were reached -20 minutes after application of the donor systems. It takes 2 hours for uncoated aspirin tablets to reach the peak salicylate level and much longer for coated aspirin tablets when they are taken orally. The peaks were ~0.01mg/ml for salicylic acid and ~10 mg/ml for diethylaminoethyl acetylsalicylate.AcOH (approximately 100 times difference). ~10 mg/ml of salicylic acid in plasma is more than 33-67 times higher than the salicylate plasma level (0.15-0.3 mg/ml) for effective analgesia and 33-50 times higher than the salicylate plasma level (0.2-0.3 mg/ml) for effective anti-inflammatory activity. This is a very exciting result. It will be very easy and fast to deliver therapeutically effective plasma levels of aspirin into the host by these pro-drugs. These results suggest that the pro-drugs can be administered not only orally, but also transdermally for any kind of medical treatments. The in vivo rates of penetration of other prodrugs of the general "Structure 1" are close to that of diethylaminoethyl acetylsalicylate.AcOH.

To check the gastroduodenal bleeding caused by drugs, the rats (two groups, each group had 10 rats) were orally administered with 100 mg/kg of aspirin or diethylaminoethyl acetylsalicylate.AcOH per day for 21 days. We found an average of 5 mg of fecal blood per gram of feces in the aspirin group and none in diethylaminoethyl acetylsalicylate.AcOH group.

The acute toxicity of the pro-drugs were investigated. The $LD_{50}$ orally in rats are: 2.3 g/kg and 2.2 g for diethylaminoethyl acetylsalicylate.AcOH and dimethylaminoethyl acetylsalicylate AcOH. The results show that the pro-drugs are less toxic than aspirin ($LD_{50}$=1.5g/kg).

Acetylsalicylic acid has demonstrated anti-inflammatory, analgesic, antipyretic, and antirheumatic activity, inhibits platelet aggregation, reduces cardiac mortality , but salicylic acid has only analgesic and antipyretic activity.

A good pro-drug should go back to the drug itself in plasma. Diethylaminoethyl acetylsalicylate.AcOH has two ester function groups. One (acetyl) is from acetylsalicylic acid itself, and the one is the diethylaminoethyl ester (the added one). Both ester groups can be rapidly cleaved by the enzymes in human plasma in vitro. If the acetate group losses faster than diethylaminoethyl, the pro-durg will be changed to diethylaminoethyl salicylate, then to salicylic acid instead of acetylsalicylic acid. Diethylaminoethyl acetylsalicylate.AcOH and Acetylsalicylic acid have very short half-lives (-4 min. and 15 min.) in whole human blood. To determine how much the pro-drugs of acetylsalicylic acid were changed back to the drug itself or diethylaminoethyl salicylate, we diluted whole human blood 20 times with a pH 7.4 phosphate

buffer (0.2M). The cumulative amounts of diethylaminoethyl salicylate, acetylsalicylic acid, and salicylic acid were determined by a specific high-performance liquid chromatography method. The results shown the rate of diethylaminoethyl salicylate vs acetylsalicylic acid is 1 vs 4. This means that 80% of the pro-drug was changed back to the drug itself. Due to the pro-drug having a much better absorption rate, the pro-drug will have more strength than the acetylsalicylic acid itself at the same dosage.

The analgetic, antipyretic, and anti-inflammatory activities of diethylaminoethyl acetylsalicylate.AcOH were tested using aspirin as a comparison. Other compounds of of the general "Structure 1 " were tested by the same methods and have very similar results as that of diethylaminoethyl acetylsalicylate.AcOH .

Analgetic activity: The prolongation time of pain the threshold of a mouse tail was determined in accordance with the D'Amour-Smith Method (J. Pharmacol. Exp. Ther., 72, 74(1941)). After 200mg/kg of aspirin was administered orally and 200mg/kg of diethylaminoethyl salicylate.AcOH was administered orally and transdermally, the tails of mice were exposed to heat and the prolongation time of pain threshold was determined. The results obtained are shown in Figure 3. In Figure 3, the groups administered 200 mg/kg of diethylaminoethyl salicylate.AcOH orally (C) and transdermally (D) were shown to exhibit stronger analgetic activity than the group administered 200mg/kg of aspirin.

The number of writhings that occurred when mice were administered an acetic acid solution intraperitoneally were counted, and the rate of inhibition based on the control group was calculated. 42 mice were divided into 7 groups (6 mice each). Aspirin (ASA, 50 mg/kg and 100 g) was administered to groups B1 and B2 of mice and diethylaminoethyl acetylsalicylate.AcOH (DEAE-ASA, 50 mg and 100 mg/kg) was administered orally to groups C1 and C2. Diethylaminoethyl acetylsalicylate.AcOH (DEAE-ASA, 50 mg and 100 mg/kg) was administered transdermally to groups D1 and D2. The A group is the control group. The test compounds were administered to the mice 30 minutes before the acetic acid solution was administered. The results are shown in following Table 1.

Table 1. The rate of writhings inhibition by and aspirin and its pro-drugs

| Group | A | B1 | B2 | C1 | C2 | D1 | D2 |
|---|---|---|---|---|---|---|---|
| Dose (mg/kg) | 0 | 50 | 100 | 50 | 100 | 50 | 100 |
| No. of Writhings | 33.2 | 18.2 | 13.2 | 15.4 | 11 | 14.5 | 10.1 |
| % | - | 45 | 60 | 54 | 67 | 56 | 70 |

The results show that diethylaminoethyl acetylsalicylate.AcOH demonstrates better analgetic activity than aspirin. Other compounds of the general "Structure 1" show similar analgetic activity.

Antipyretic activity: Rats received a sterilized E. coli suspension as a pyrogen. 56 rats were divided into 7 groups. The control group is group A. 2 hours later, Aspirin (ASA, B1 for 100mg/kg and B2 for 150mg/kg) and diethylaminoethyl acetylsalicylate.AcOH (DEAE-ASA, C1 for 100mg/kg and C2 for 150 mg) were administered orally and diethylaminoethyl acetylsalicylate.AcOH (DEAE-ASA, D1 for 100 mg and D2 for 150 mg/kg) were administered transdermally. The body temperature of rats was taken at 90 min. intervals before and after the administration of the test compounds. The results are shown in the following Table 2.

Table 2. Antipyretic activity of aspirin and its pro-drugs

| Compound | t=0 min | t=90 min | t=180 min. | t=270 min. |
|---|---|---|---|---|
| Control group(A) | 37.32±0.05 | 37.35±0.04 | 37.12±0.05 | 37.08±0.04 |
| ASA (100mg/kg, B1) | 37.22±0.05 | 36.80±0.05 | 36.85±0.05 | 36.81±0.05 |
| ASA (150mg/kg, B2) | 37.30±0.06 | 36.50±0.05 | 36.59±0.05 | 36.55±0.05 |
| DEAE-ASA (100mg/kg, C1, orally) | 37.25±0.09 | 36.40±0.15 | 36.50±0.09 | 36.40±0.15 |
| DEAE-ASA (150mg/kg, C2, orally) | 37.18±0.07 | 36.30±0.15 | 36.28±0.07 | 36.20±0.09 |
| DEAE-ASA (100mg/kg, D1, transdermally) | 37.19±0.07 | 36.40±0.05 | 36.38±0.05 | 36.40±0.15 |
| DEAE-ASA (150mg/kg, D2, transdermally) | 37.33±0.05 | 36.27±0.15 | 36.26±0.07 | 36.22±0.08 |

The results shown that diethylaminoethyl acetylsalicylate.AcOH demonstrated antipyretic activity at 100 mg/kg dose and some better than aspirin. The results show that transdermal administration of diethylaminoethyl acetylsalicylate.AcOH is better than oral administration. Other compounds of the general "Structure 1" show similar antipyretic activity.

Anti-inflammatory activity: 50 mg/kg of diethylaminoethyl acetylsalicylate.AcOH was administered orally or transdermally to rats and 50 mg/kg of aspirin was administered orally. 60 minutes later, a carrageenin solution was administered subcutaneously to the foot pads of the rats. The volume of the hind paw was measured at every hour after the administration of the carrageenin, and the rate of increase in the volume of the paw was calculated and designated as the rate of swelling(%). The results obtained are shown in Figure 4.

The results show that diethylaminoethyl acetylsalicylate.AcOH demonstrated better Anti-inflammatory activity than that of aspirin at 50mg/kg for oral administration and transdermal administration. Other compounds of the general "Structure 1" shown similar anti-inflammatory activity.

It is also known that a high dose of oral acetylsalicylic acid shows an anti-reactive-antiasthmatic activity by inhibition of the cyclooxygenase activity (Bianco, Sebastiano, U.S. Pat. No. 5,570,559), Due to their very high membrane penetration rate, these pro-drugs can be used in treating asthma by spraying into the mouth or nose of a host. They can also be used to treat acne due to their anti-inflammatory properties. They can be used for the treatment and prevention of endothelia dysfunction as well.

The compounds of the general formula(1) "Structure 1" indicated above can be prepared from ASA or its analogues, or from functional derivatives of ASA or its analogues. For example, acid halides or mixed anhydrides of the general formula(2) "Structure 2".

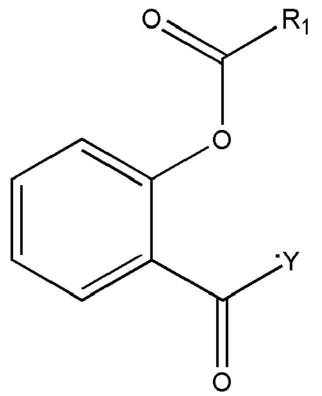

Structure 2

In which $R_1$ represents $CH_3$, $C_2H_5$, $C_3H_7$, or other lower alkyl groups; Y represents halogen, alkoxycarbonyl or substituted aryloxycarbonyloxy, by reaction with compounds of the general formula (3) "Structure 3".

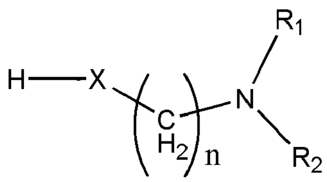

Structure 3

In which, R represents H, one of any alkyl, alkyloxy, or alkenyl residues having 1 to 6 carbon atoms, or aryl residues; $R_2$ represents H, one of any alkyl, alkyloxy, or alkenyl residues having 1 to 6 carbon atoms, or aryl residues; X represents O, S or NH; and n=0,1,2,3,4,5.....

The compounds of the general "Structure 1" indicated above can be prepared from ASA or its analogues, by reaction with compounds of the general formula (3) "Structure 3" by using coupling reagents, such as N,N'-Dicyclohexylcarbodiimide , N,N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluoro phosphate, Benzotriazol-1-yl-oxy-tris(dimethylamino)phosphonium hexafluorophosphate, et al.

When X represents O, the compounds of the general formula(1) "Structure 1" indicated above can be prepared from metal salts or organic base salts of acetylsalicylic acid or its analogues, by reaction with compounds of the general formula (4) "Structure 4".

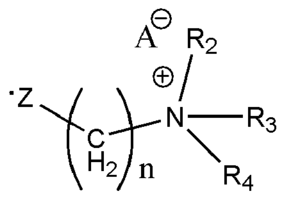

Structure 4

In which, $R_2$ represents H, one of any alkyl, alkyloxy, or alkenyl residues having 1 to 6 carbon atoms, or aryl residues; $R_3$ represents H, one of any alkyl, alkyloxy, or alkenyl residues having 1 to 6 carbon atoms, or aryl residues; $R_4$ represents H, one of any alkyl, alkyloxy, or alkenyl residues having 1 to 6 carbon atoms, or aryl residues; Z represents halogen, or p-toluenesulphonyl, $A^-$ represents $Cl^-$, $Br^-$ $F^-$, $I^-$, $AcO^-$, acetylsalicylate, citrate, salicylate, or any negative ions; and n=0,1,2,3,4,5.....

When X represents O, the compounds of the general "Structure 1" indicated above can be prepared from immobilized base salts of acetylsalicylic acid or its analogues of the general formula (5) "Structure 5".

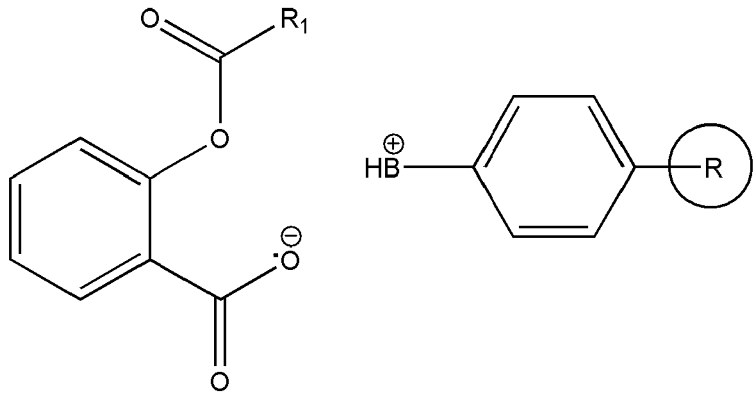

Structure 5

In which, R represents cross-linked resin; $R_1$ represents $CH_3$, $C_2H_5$, $C_3H_7$, or other lower alkyl group; B represents any base groups, such as pyridine, piperidine, triethylamine, or other base groups, by reaction with compounds of the general formula(4) "Structure 4".

The present invention relates to pharmaceutical preparations comprising pro-drugs of acetylsalicylic acid and its analogues of the general formula(1) "Structure 1" in addition to customary auxiliaries and excipients, e.g. in the form of tablets, capsules or solutions for administration orally and in the form of solutions, lotion, ointment, emulsion or gel for transdermal administration transdermally. The new active compounds of the general "Structure 1" can be combined with vitamins such as A,B,C or E or beta-carotene, or other pharmaceuticals, such as folic acid, et. al for treating any aspirin-treatable conditions in humans or animals.

Transdermal therapeutic application systems of compounds of the general "Structure 1" or a composition comprising at least one compound of the general formula(1) "Structure 1 ", as an active ingredient, for treating any aspirin-treatable conditions in humans or animals, especially for preventing thrombosis and other cardiovascular diseases, and cancer prophylaxis can be developed. These systems can be a bandage or a patch comprising one active substance-containing matrix layer and an impermeable backing layer. The most preferable system is an active substance reservoir, which has a permeable bottom facing the skin. By controlling the rate of release, this system enables aspirin to reach constantly optimal therapeutic blood levels to increase effectiveness and reduce the side effects of aspirin. These systems can be worn on wrist, ankle, arm, leg, or any part of body.

**Advantageous Effects**

These pro-drugs of aspirin have a lipophilic portion and a hydrophilic portion (the amine groups that exist in the protonated form at physiologic pH). The positively charged amino groups of these pro-drugs have two major advantages. The first, it largely increases the solubility of the drugs; when these new pro-drugs are administered orally in a

dosage form such as a tablet, capsule, solution, or suspension, they will dissolve in gastric juice immediately. The second, the positive charge on the amino group of these pro-drugs will bond to the negative charge on the phosphate head group of membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semiliquid concentrated aqueous solution or suspension. Due to the short stay in the GI tract, the pro-drugs will not cause gastric mucosal cell damage. Experiment results show that 80% of the pro-drug was changed back to the drug itself. The pro-drugs have a much better absorption rate, and thus the pro-drugs will have better strength than the acetylsalicylic acid itself at the same dosage.

The experiment results suggest that the pro-drug, diethylaminoethyl acetylsalicylate.AcOH, diffuses through human skin -400 times faster than acetylsalicylic acid itself and ~100 times faster than ethyl acetylsalicylate. The in vivo rates of penetration of diethylaminoethyl acetylsalicylate.AcOH through the skin of intact hairless mice was very high. It takes 2 hours for uncoated aspirin tablets to reach the peak salicylate plasma level and much longer for coated aspirin tablets when they are taken orally, but diethylaminoethyl acetylsalicylate.AcOH only took about 20 minutes to reach the peak salicylate plasma level. The most exciting result is that the pro-drugs can be administered not only orally, but also transdermally for any type of medical treatment and should avoid most of the side effects of aspirin, most notably GI disturbances such as dyspepsia, gastroduodenal bleeding, gastric ulcerations, and gastritis.

**Description of Drawings**

Figure 1: Cumulative amounts of acetylsalicylic acid (ASA), ethyl acetylsalicylate(E-ASA), and diethylaminoethyl acetylsalicylate.AcOH(DEAE-ASA) crossing isolated human skin tissue in Franz cells (n=5). ASA and E-ASA were applied as 20% suspensions. DEAE-ASA was applied as a 20% solution. In each case, the vehicle was pH 7.4 phosphate buffer (0.2 M).

Figure 2: Total plasma levels of salicylic acid (SA) after topical application of 300 mg of acetylsalicylic acid (ASA) or diethylaminoethyl acetylsalicylate.AcOH (DEAE-ASA) to the backs of hairless mice (n=5).

Figure 3. The prolongation time of the pain threshold of the mice tails after 200mg/kg of aspirin (B) was administered orally, 200mg/kg of diethylaminoethyl salicylate.AcOH was administered orally (C) and transdermally (D). A is the control line.

Figure 4. The rate of swelling (%) after a carrageenin injection. 1 hour before the carrageenin injection, 100 mg/kg of spirin was administered orally (B), 100 mg/kg of diethylaminoethyl salicylate.AcOH (C) was administered orally and transdermally (D). A is the control line.

Structure 1. In which, $R_1$ represents $CH_3$, $C_2 H_5$, $C_3 H_7$, or other lower alkyl groups; $R_2$ represents H, one of any alkyl, alkyloxy, or alkenyl residues having 1 to 6 carbon atoms, or aryl residues; $R_3$ represents H, one of any alkyl, alkyloxy, or alkenyl residues having 1 to 6 carbon atoms, or aryl residues; R 4 represents H, one of any alkyl, alkyloxy, or alkenyl residues having 1 to 6 carbon atoms, or aryl residues; X represents O, S or NH; $A^-$ represents $Cl^-$, $Br^-$, F, $I^-$, $AcO^-$, acetylsalicylate, citrate, salicylate, or any negative ions; and n=0,1,2,3,4,5......

**Best Mode**

Preparation of diethylaminoethyl acetylsalicylate.AcOH

18 g (0.1 mol) of o-acetylsalicylic was dissolved in 180 ml of chloroform. 12.5 g of Sodium bicarbonate (0.15 mol) was added into the solution. Water (20 ml) was added with stirring. After the mixture had been stirred for 30 minutes, anhydrous sodium sulfate (200 g) was added. 39g (0.15 mol) of diethylaminoethyl bromide.HBr was added into the mixture and the mixture was stirred for 5 hours at RT. 8.2 g (0.1 mol) of sodium acetate was added into the reaction mixture with stirring. The mixture is stirred for 2 hours. The solid was removed by filtration and washed with chloroform (3 x 50 ml). The solution is concentrated in vacuo to 100 ml. Then 300 ml of hexane was added into the solution. The solid product was collected by filtration and washed with hexane (3 x 100 ml). After drying, it yielded 31 g of the desired product (91 %). Hygroscopic product; Solubility in water: 300 mg/ml; Elementary analysis: $C_{17} H_{25} NO_6$; MW: 339.38. Calculated % C: 60.07; H: 7.44; N: 4.15; O: 28.22 ; Found % C: 60.16; H: 7.42; N: 4.13; O: 28.29. $^1$H-NMR(400 MHz, $CDCL_3$): delta: 1.55(t, 6H), 2.08(s, 3H), 2.20(s, 3H), 3.28(m, 4H); 3.70(m,2H), 4.68(m, 2H), 6.5(b, 1H), 7.17(m, 1H), 7.19 (m,1H),7.45(m,1H),7.94(m, 1H).

**Mode for Invention**

Preparation of dimethylaminoethyl acetylsalicylate.AcOH

19.9 g (0.1 mol) of o-acetoxybenzoyl chloride was dissolved in 100 ml of chloroform. The mixture was cooled to 0°C. 15 ml of triethylamine and 8.9 g of dimethylaminoethanol were added into the reaction mixture. The mixture is stirred for 3 hours at RT. 6 g of acetic acid is added into the reaction mixture with stirring. The so lid side product was removed by filtration and washed with chloroform (3 x 30 ml). The organic solution was evaporated off. After drying, it yielded 29 g of the desired product (93%). Hygroscopic product; Solubility in water: 350 mg/ml; Elementary analysis: $C_{15}$ $H_{21}$ $NO_6$; MW: 311.33. Calculated % C: 57.87; H: 6.80; N: 4.50; O: 30.83 ; Found % C: 57.82; H: 6.85; N: 4.48; O: 30.85. $^1$H-NMR (400 MHz, $CDCL_3$): delta: 2.09 (s, 3H) 2.21 (s, 3H), 2.90 (s, 6H); 3.71(m,2H), 4.69 (m, 2H), 6.9 (b, 1H), 7.18 (m, 1H), 7.20 (m,1H),7.47(m,1H),7.93 (m, 1H)

Preparation of S-dimethylaminoethyl acetylthiosalicylate.AcOH

19.9 g (0.1 mol) of o-acetoxybenzoyl chloride was dissolved in 100 ml of chloroform. The mixture was cooled to 0°C. 15 ml of triethylamine and 9.3 g of dimethylaminoethyl mercaptan were added into the reaction mixture. The mixture was stirred for 3 hours at RT. 6 g of acetic acid was added into the reaction mixture with stirring. The solid side product was removed by filtration and washed with chloroform (3 x 30 ml). The organic solution was evaporated off. After drying, it yielded 28 g of the desired product (87%). Hygroscopic product; Solubility in water: 320 mg/ml; Elementary analysis: $C_{15}$ $H_{21}$ $NO_5$ S; MW: 327.4. Calculated % C: 55.03; H: 6.47; N: 4.28; O: 24.43 S:9.79 ; Found % C: 55.02; H: 6.45; N: 4.35; O: 24.49; 9.69. $^1$H-NMR (400 MHz, $CDCL_3$): delta: 2.09 (s, 3H) 2.21 (s, 3H), 2.90 (s, 6H); 3.31 (t,2H), 3.91 (m, 2H), 6.9 (b, 1H), 7.26 (m, 1H), 7.28 (m,1H),7.55 (m,1H),7.94 (m, 1H)

Preparation of N-dimethylaminoethyl acetylsalicylamide.AcOH

19.9 g (0.1 mol) of o-acetoxybenzoyl chloride was dissolved in 100 ml of chloroform. The mixture was cooled to 0°C. 15 ml of triethylamine and 8.9 g of dimethylaminoethylamine were added into the reaction mixture. The mixture was stirred for 3 hours at RT. 6 g of acetic acid was added into the reaction mixture with stirring. The solid side product was removed by filtration and washed with chloroform (3x30 ml). The organic solution was evaporated off. After drying, yielded 28 g of the desired product(90.2%). Hygroscopic product; Solubility in water: 350 mg/ml; Elementary analysis: $C_{15}H_{22}N_2O_5$; MW: 310.35. Calculated % C: 58.05; H: 7.15; N: 9.03; O: 25.78 ; Found % C: 58.02; H: 7.18; N: 8.98; O: 25.83. $^1$H-NMR (400 MHz, $CDCL_3$): delta: 2.09 (s, 3H) 2.21(s, 3H), 2.90 (s, 6H); 3.54 (m,2H), 3.64 (t, 2H), 6.9 (b, 1H), 7.8 (b, 1H); 7.25 (m, 1H), 7.26 (m,1H),7.48 (m,1H), 7.92 (m, 1H).

Preparation of S-diethylaminoethyl propionylthiosalicylate.AcOH

18 g (0.1 mol) of o-acetylsalicylic acid was dissolved in 100 ml of dichloromethane (DCM). The mixture was cooled to 0°C. 20.6 g of 1,3-Dicyclohexylcarbodiimid was added into the reaction mixture. The mixture was stirred for 30 minutes at 0°C. 14.8 g (0.1 mol) of diethylaminopropyl mercaptan was added into the reaction mixture. The mixture was stirred for 3 h at RT. 6 g of acetic acid was added into the reaction mixture with stirring. The solid side product was removed by filtration and washed with chloroform (3 x 50 ml). The organic solution was evaporated off. After drying, it yielded 32 g of the desired product (86.6%). Hygroscopic product; Solubility in water: 280 mg/ml; Elementary analysis: $C_{18}$ $H_{27}$ $NO_5$ S; MW: 369.48. Calculated % C: 58.51; H: 7.37; N: 3.79; O:21.65 ;S:8.68 Found % C: 58.53; H: 7.39; N: 3.75; O: 21.68; S: 8.65. $^1$H-NMR (400 MHz, $CDCL_3$): delta: 1.09(t, 3H),1.56 (t, 6H), 2.21 (s, 3H),2.27 (m, 2H) 3.28 (m, 4H), 3.31 (m, 2H); 3.91 (m,2H), 6.8 (b, 1H), 7.25 (m, 1H), 7.26 (m,1H), 7.48 (m,1H), 7.92 (m, 1H)

Preparation of N-diethylaminopropyl acetylsalicylamide.AcOH

18 g (0.1 mol) of o-acetylsalicylic acid was dissolved in 100 ml of acetonitrile. 32.1 g of O-(Benzotriazol-1-yl)-N, N,N',N'-tetramethyluronium tetrafluoroborate and 30 ml of triethylamine were added into the reaction mixture. 13.1 g of dimethylaminopropylamine was added into the reaction mixture. The mixture was stirred for 3 hours at RT. The solvents were evaporated off. 250 ml of ethyl acetate was added into the reaction mixture and the mixture was washed with water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 32 g of the desired product(90.8%).Hygroscopic product; Solubility in water: 280 mg/ml; Elementary analysis: $C_{18}H_{28}N_2O_5$;MW: 352.43. Calculated % C: 61.34; H: 8.01; N: 7.95; O:22.70 ; Found % C: 61.25; H: 8.05; N: 7.96; O: 22.74. $^1$H-NMR (400 MHz, $CDCL_3$): delta: 1.56 (t, 6H) 2.03 (m, 2H) 2.09 (s, 3H), 2.21(s, 3H), 3.24 (m, 2H), 3.20 (m, 2H); 3.24 (m,2H), 6.9 (b, 1H), 7.8 (b, 1H); 7.25 (m, 1H), 7.26 (m,1H),7.48 (m,1H),7.92 (m, 1H).

Preparation of dipropylaminoethyl acetylsalicylate.AcOH

20.3 g (0.1 mol) of sodium o-acetylsalicylate was suspended in 180 ml of chloroform. 28.8 g (0.1 mol) of dipropylaminoethyl bromide.HBr was added into the mixture and the mixture was stirred for 5 hours at RT. 8.2 g (0.1 mol) of sodium acetate was added into the reaction mixture with stirring. The mixture is stirred for 2 hours. The solid was removed by filtration and washed with chloroform (3 x 50 ml). The solution is concentrated in vacuo to 100 ml. Then 300 ml of hexane was added into the solution. The solid product was collected by filtration and washed with hexane (3 x 100 ml). After drying, it yielded 30 g of the desired product (81.6%). Hygroscopic product; Solubility in water: 300 mg/ml; Elementary analysis: $C_{17}H_{25}NO_6$; MW: 367.44. Calculated % C: 62.11; H: 7.96; N: 3.81; O: 26.13 ; Found % C: 62.07; H: 7.99; N: 3.78; O: 26.17. $^1$H-NMR (400 MHz, $CDCL_3$): delta: 0.97 (t, 6H),1.77 (m, 4H), 2.20 (s, 3H), 3.25 (m, 4H); 3.70 (m,2H), 4.69 (m, 2H), 6.8 (b, 1H), 7.17 (m, 1H), 7.19 (m,1H),7.45 (m,1H),7.94 (m, 1H).

Preparation of dipropylaminoethyl acetylsalicylate.AcOH

60 g of Polymer-bound triethylamine (3 mmol/g, 100-200 mesh) was suspended in 180 ml of chloroform. 18 g (0.1 mol) of o-acetylsalicylic acid was added into the mixture with stirring. 43 g (0.15 mol) of dipropylaminoethyl bromide.HBr was added into the mixture and the mixture was stirred for 5 hours at RT. The polymer is removed by filtration and washed with tetrahydrofuran (3 x 50 ml). 8.2 g (0.1 mol) of sodium acetate was added into the reaction mixture with stirring. The mixture is stirred for 2 h. The solid was removed by filtration and washed with chloroform (3 x 50 ml). The solution is concentrated in vacuo to 100 ml. Then 300 ml of hexane was added into the solution. The solid product was collected by filtration and washed with hexane (3 x 100 ml). After drying, it yielded 31 g of the desired product (91 %). Hygroscopic product; Solubility in water: 300 mg/ml; Elementary analysis: $C_{17}H_{25}NO_6$; MW: 339.38. Calculated % C: 60.07; H: 7.44; N: 4.15; O: 28.22 ; Found % C: 60.16; H: 7.42; N: 4.13; O: 28.29. $^1$H-NMR (400 MHz, $CDCL_3$): delta: 1.55 (t, 6H), 2.08 (s, 3H), 2.20 (s, 3H), 3.28 (m, 4H); 3.70 (m,2H), 4.68 (m, 2H), 6.5 (b, 1H), 7.17 (m, 1H), 7.19 (m,1H), 7.45 (m,1H),7.94 (m, 1H).

## Industrial Applicability

The pro-drugs of of the general "Structure 1" are superior to aspirin. They may be used medicinally in treating any aspirin-treatable conditions in humans or animals. They may be used in the long-term palliative treatment of mild to moderate pain and inflammation of arthritis and other inflammatory conditions. They may be used alone or as an adjunct in the treatment of Kawasaki syndrome, thromboembolism after surgery, and unstable angina. They can be used to decrease stool volume and increase weight in acute childhood gastroenteritis, as an adjunct to prevent aortocoronary-artery -bypass graft occlusion, and to prevent thromboembolic complications in chronic artrial fibrillation. They can be used as an adjunct in carotid endarterectomy to reduce platelet aggregation and thromboxane suppression, and they may be prescribed to reduce the development of cataracts, to prevent recurrence of stenosis after coronary angioplasty, and to improve cognitive performance and cerebral blood flow in patients with multi-infract dementia. They may be used as an adjunct to lower plasma glucose in diabetes mellitus and diabeties-induced complications, including diabetic retinopathy, necrobiotic ulcers, and diabetic proteinuria, and to decrease total and cardiovascular mortality. They can be prescribed to reduce the incidence of hemodialysis shunt thrombosis, to decrease the deterioration of renal function and occurrence of end-stage renal disease in patients with type-1 membranoproliferative glomerulonephritis, and to slow the progression of peripheral occlussive arterial disease. They may be used in the prevention of colon cancer and rectal cancer, and arterial embolic complications in patients' prosthetic heart valves. They can also be prescribed to lower the incidence of pregnancy-induced hypertension and preeclamptic toxemia in high-risk women. It is also known that high dose of oral acetylsalicylic acid shows an antireactive activity by the inhibition of the cyclooxygenase activity (Bianco, Sebastiano, U.S. Pat. No. 5,570,559). Due to their very high membrane penetration rate, these prodrugs can be used in treating asthma by inhalation to a host. They can be used to treat acne due to their anti-inflammatory properties. They can be used for the treatment and prevention of endothelial dysfunction.

## Sequence List Text

Further preferred embodiments of the present invention are given in the following paragraphs:

[1] The compounds of the general formula (1) "Structure 1".

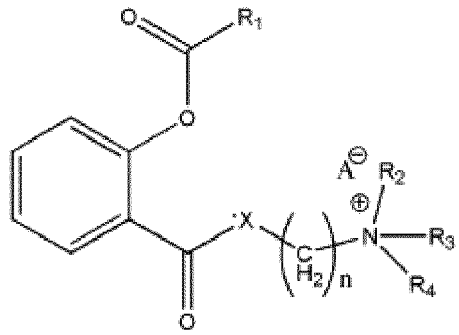

Structure 1

In which, $R_1$ represents $CH_3$, $C_2H_5$, $C_3H_7$, or other lower alkyl groups; $R_2$ represents H, one of any alkyl, alkyloxy, or alkenyl residues having 1 to 6 carbon atoms, or aryl residues; $R_3$ represents H, one of any alkyl, alkyloxy, or alkenyl residues having 1 to 6 carbon atoms, or aryl residues; $R_4$ represents H, one of any alkyl, alkyloxy, or alkenyl residues having 1 to 6 carbon atoms, or aryl residues; X represents O, S or NH; $A^-$ represents $Cl^-$, $Br^-$, $F^-$, $I^-$, $AcO^-$, acetylsalicylate, oxalate, citrate, salicylate, or any negative ions; and n=0,1,2,3,4,5....

[2] Process for the preparation of compounds of the general formula(1) "Structure 1" according to Embodiment 1.

[3] Compounds of the general formula(1) "Structure 1" or a composition comprising at least one compound of the general formula(1) "Structure 1", as an active ingredient, according to Embodiment 1, where they can be administered orally or transdermally, for treating any aspirin-treatable conditions in humans or animals . The aspirin-treatable conditions include, but are not limited to, pain from a toothache, headache, and arthritis and other inflammatory pain, fever, preeclampsia, heart attacks, Kawasaki syndrome, thromboembolism after surgery, and unstable angina, acute childhood gastroenteritis, aortocoronary-artery - bypass graft occlusion, thromboembolic complications in chronic artrial fibrillation, cataracts, platelet aggregation and thromboxane suppression, recurrence of stenosis after coronary angioplasty, diabetes mellitus and diabetic-induced complications, including diabetic retinopathy, necrobiotic ulcers, and diabetic proteinuria, the incidence of hemodialysis shunt thrombosis, the deterioation of renal function and occurrence of end-stage renal disease in patients with type-1 membranoproliferative glomerulonephritis, progression of peripheral occlussive arterial disease, the prevention of colon cancer and rectal cancer, and arterial embolic complications in patients prosthetic heart valves.

[4] A method for treating any aspirin-treatable conditions in humans or animals by administering transdermally to any part of body (in the form of a solution, spray, lotion, ointment, emulsion or gel) to deliver therapeutically effective plasma levels of compounds of the general formula(1) "Structure 1" or a composition comprising at least one compound of the general formula(1) "Structure 1", as an active ingredient, according to Embodiment 1.

[5] A method for topically treating pain such as a headache, toothache, and muscle pain, and arthritis and other inflammatory pain in humans or animals by administering to the inflamed area a therapeutically effective amount of the compounds of the general formula(1) "Structure 1" or a composition comprising at least one compound of the general "Structure 1", as an active ingredient, according to Embodiment 1.

[6] Compounds of the general formula(1) "Structure 1" or a composition comprising at least one compound of the general "Structure 1", as an active ingredient, according to Embodiment 1, may be administered transdermally, for treating acne or other skin disorders in the form of a solution, spray, lotion, ointment, emulsion or gel.

[7] Compounds of the general formula(1) "Structure 1" or a composition comprising at least one compound of the general "Structure 1", as an active ingredient, according to Embodiment 1, are administered by spraying to through the mouth or nose or other parts of body for treating asthma.

[8] Transdermal therapeutic application systems of Compounds of the general formula(1) "Structure 1" or a composition comprising at least one compound of the general "Structure 1", as an active ingredient, according to embodiment 1 for treating any aspirin-treatable conditions in humans or animals, especially for preventing thrombosis

and other cardiovascular diseases, and cancer prophylaxis. These systems can be a bandage or a patch comprising one active substance- containing matrix layer and an impermeable backing layer. The most preferable system is an active substance reservoir, which has a permeable bottom facing the skin. By controlling the rate of release, this system enables the aspirin to reach constantly optimal therapeutic blood levels to incease effectiveness and reduce the side effects of aspirin

## Claims

1. A compound having a general formula of Structure 1:

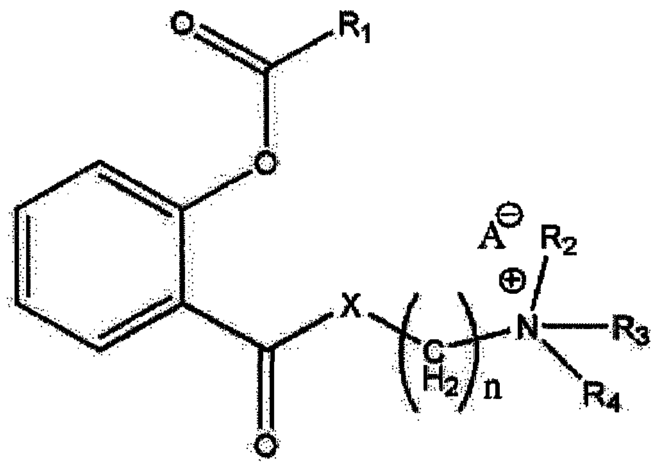

Structure 1,

or a composition comprising at least one compound having a general formula of Structure 1 as an active ingredient, wherein:

$R_1$ is selected from the group consisting of $CH_3$, $C_2H_5$, $C_3H_7$, or other lower alkyl;
$R_2$ is selected from the group consisting of H, alkyl residues having 1 to 6 carbon atoms, alkyloxy residues having 1 to 6 carbon atoms, alkenyl residues having up to 6 carbon atoms, and aryl residues;
$R_3$ is selected from the group consisting of H, alkyl residues having 1 to 6 carbon atoms, alkyloxy residues having 1 to 6 carbon atoms, alkenyl residues having up to 6 carbon atoms, and aryl residues;
$R_4$ is selected from the group consisting of H, alkyl residues having 1 to 6 carbon atoms, alkyloxy residues having 1 to 6 carbon atoms, alkenyl residues having up to 6 carbon atoms, and aryl residues;
X represents O, S, or NH;
$A^-$ represents $Cl^-$, $Br^-$, $F^-$, $I^-$, $AcO^-$, acetylsalicylate, oxalate, citrate, salicylate, or any negative ion; and

$$n = 0, 1, 2, 3, 4, 5.$$

2. The composition according to claim 1, wherein the composition further comprises water.

3. The compound or composition according to claim 1 or 2 for use in a method of treatment of any aspirin-treatable conditions in humans or animals, wherein the compound or composition is administered orally or transdermally.

4. The compound or composition for use according to claim 3, wherein the aspirin-treatable condition is selected from the group consisting of pain from a toothache, pain from a headache, pain from arthritis, other inflammatory pain, fever, pre-eclampsia, heart attacks, Kawasaki syndrome, thromboembolism after surgery, unstable angina, acute childhood gastroenteritis, aortocoronary-artery-bypass graft occlusion, thromboembolic complications in chronic artrial fibrillation, cataracts, platelet aggregation and thromboxane suppression, recurrence of stenosis after coronary angioplasty, diabetes mellitus and diabetic-induced complications, diabetic retinopathy, necrobiotic ulcers, diabetic

proteinuria, an incidence of hemodialysis shunt thrombosis, deterioation of renal function and occurrence of end-stage renal disease in patients with type-1 membranoproliferative glomerulonephritis, progression of peripheral occlusive arterial disease, prevention of colon cancer and rectal cancer, arterial embolic complications in patients prosthetic heart valves, and acne.

**5.** The compound or composition according to claim 1 or 2 for use in a method of treatment of any aspirin-treatable conditions in humans or animals, wherein the compound or composition is administered transdermally to any part of body to deliver a therapeutically effective plasma level of the compound or the composition according to claim 1 or 2.

**6.** The compound or composition for use according to claim 5, wherein the compound or composition is in the form of a solution, spray, lotion, ointment, emulsion, or gel.

**7.** The compound or composition according to claim 1 or 2 for use in a method of treatment of inflammatory pain in humans or animals, wherein the compound or composition is topically administered to the inflamed area in a therapeutically effective amount.

**8.** The compound or composition for use according to claim 7, wherein the treated condition is selected from the group consisting of headache, toothache, muscle pain, and arthritis.

**9.** The compound or composition according to claim 1 or 2 for use in a method of treatment of any aspirin-treatable conditions, wherein the compound or composition is in the form of a solution, spray, lotion, ointment, emulsion, or gel, and administered transdermally, and wherein the aspirin-treatable condition is acne or a skin disorder.

**10.** The compound or composition according to claim 1 or 2 for use in a method of treatment of any aspirin-treatable conditions, wherein the compound or composition is administered by spraying to the mouth, nose, or other parts of the body, and the aspirin-treatable condition is asthma.

**11.** A transdermal therapeutic application system comprising a compound of Structure 1 according to claim 1 or a composition comprising at least one compound of Structure 1 according to claim 1 as an active ingredient.

**12.** The transdermal therapeutic application system according to claim 11, wherein the transdermal therapeutic application system comprises a bandage or a patch comprising an active ingredient containing matrix layer and an impermeable backing layer.

**13.** The transdermal therapeutic application system according to claim 12 further comprising an active ingredient reservoir, which comprises a permeable bottom facing the skin, and by controlling the rate of release, the system enables constant optimal therapeutic blood level of aspirin in a subject the system is applied to.

**14.** The transdermal therapeutic application system according to claims 11-13 for use in a method of treatment or prevention of any aspirin-treatable conditions in a human or animal.

**15.** The transdermal therapeutic application system according to claim 14, wherein the aspirin-treatable condition is thrombosis, other cardiovascular diseases, and cancer prophylaxis.

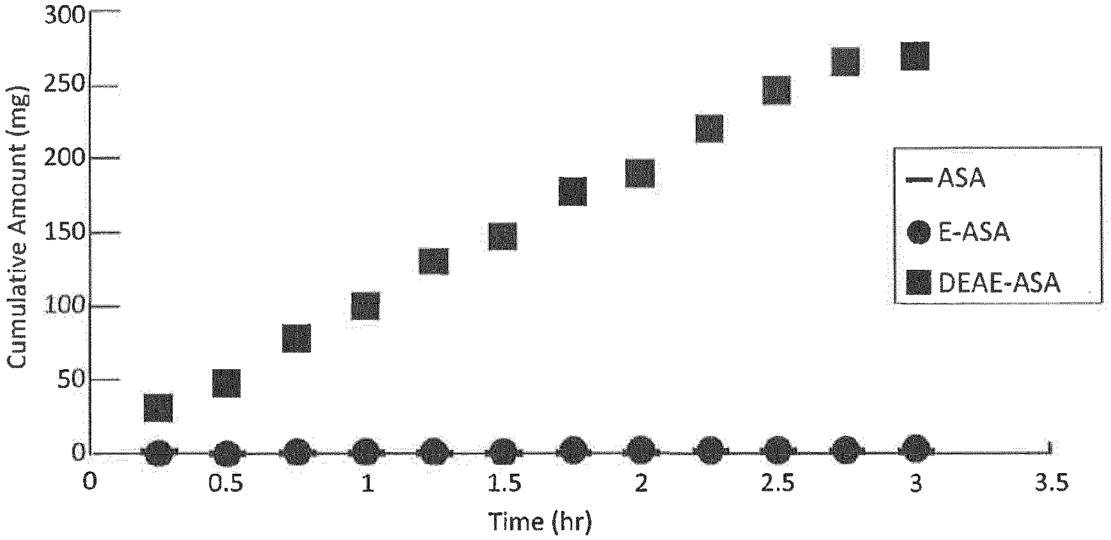


Fig. 1

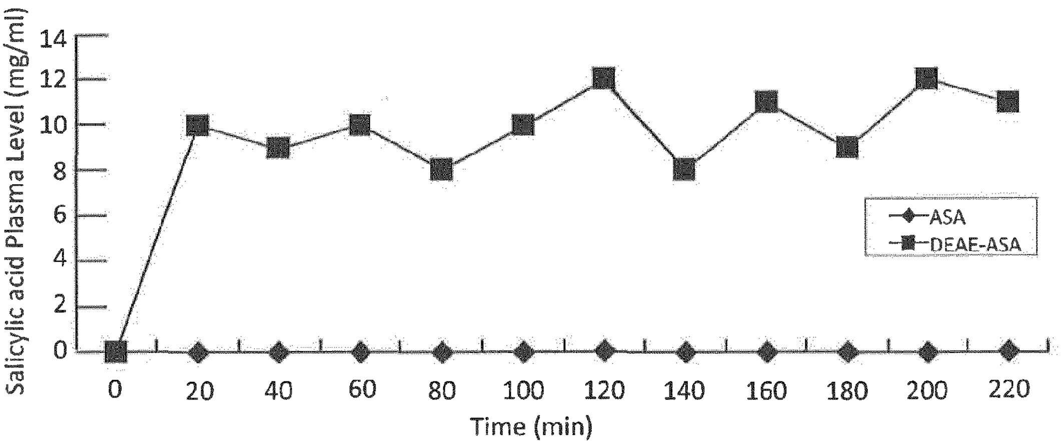


Fig. 2

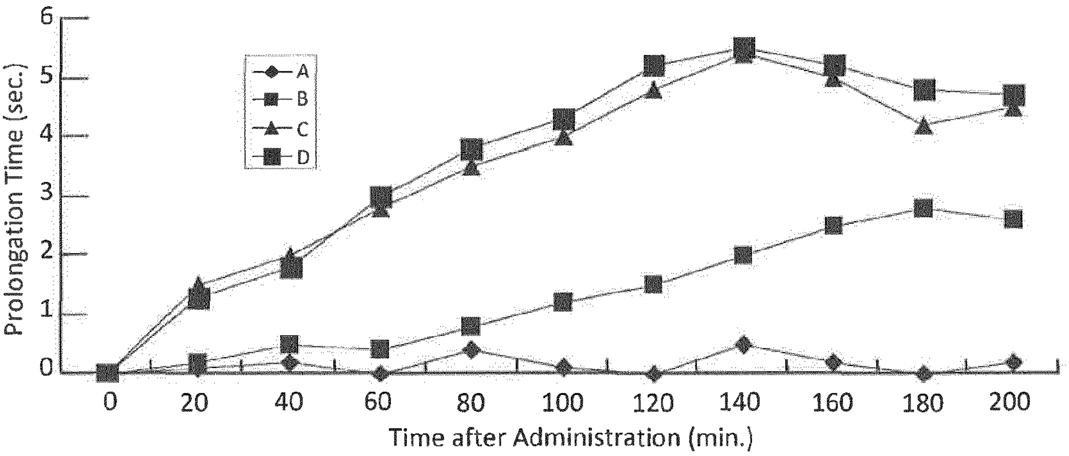


Fig. 3

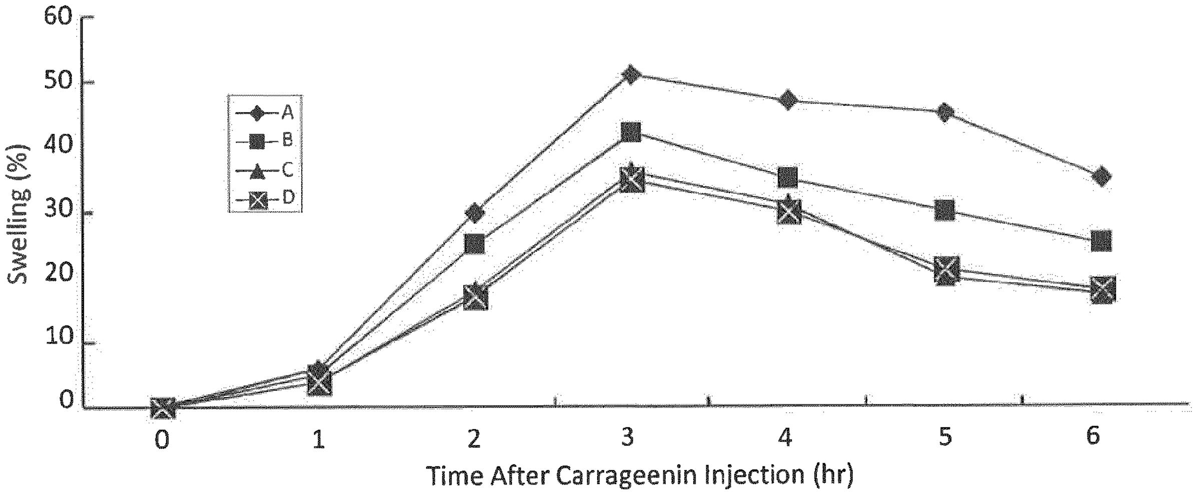


Fig. 4

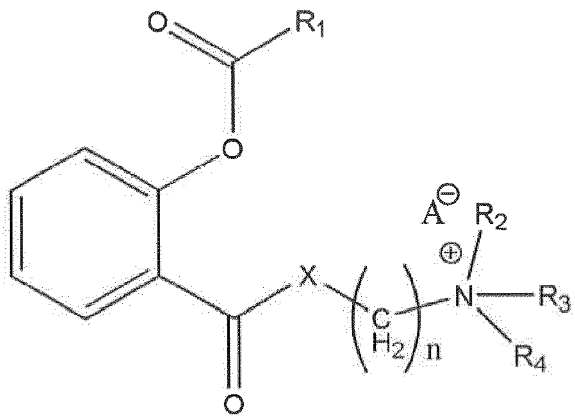

Structure 1

Fig. 5

Europäisches Patentamt
European Patent Office
Office européen des brevets

# EUROPEAN SEARCH REPORT

Application Number
EP 13 15 5198

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 152 379 A2 (CIBA GEIGY AG [CH]) 21 August 1985 (1985-08-21)<br>* page 4, paragraph 1 *<br>* page 34, paragraph 5 *<br>* claim 12 *<br>* page 10, line 14 *<br>* page 11, line 8: Edan *<br>* page 58, line 29: Edan *<br>----- | 1-15 | INV.<br>C07C219/14<br>C07C235/60<br>C07C327/30<br>A61K31/235<br>A61P29/00 |
| X | GB 958 186 A (BIOSEDRA LAB) 21 May 1964 (1964-05-21)<br>* page 1, line 13 - line 20 *<br>* page 2, line 6 - line 9 *<br>* page 2, line 35 - line 38 *<br>* page 2; example 2 *<br>----- | 1-4 | |
| X | CHANAL, J. L. ET AL: "Study on the distribution and elimination of dimethylaminoethyl acetylsalicylate in the rat. Effect of the carbon-14 labeling position", BOLLETTINO CHIMICO FARMACEUTICO , 119(6), 331-8 CODEN: BCFAAI; ISSN: 0006-6648, 1980, XP008119887,<br>* page 331 *<br>* page 332; compounds F501,F502,F503 *<br>* page 333, line 1 - line 2 *<br>-----<br>-/-- | 1-4 | TECHNICAL FIELDS SEARCHED (IPC)<br>C07C<br>A61K<br>A61P |

The present search report has been drawn up for all claims

1

| Place of search | Date of completion of the search | Examiner |
|---|---|---|
| The Hague | 29 May 2013 | Fitz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 15 5198

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | NIELSEN N M ET AL: "EVALUATION OF GLYCOLAMIDE ESTERS AND VARIOUS OTHER ESTERS OF ASPIRIN AS TRUE ASPIRIN PRODRUGS", JOURNAL OF MEDICINAL CHEMISTRY, AMERICAN CHEMICAL SOCIETY, US, vol. 3, no. 32, 1 January 1989 (1989-01-01), pages 727-734, XP001076610, ISSN: 0022-2623, DOI: 10.1021/JM00123A040 * page 729, table II, compound 25 * * page 727, column 1, paragraph 1 * * abstract * ----- | 1-4 | |
| X | DATABASE CA [Online] CHEMICAL ABSTRACTS SERVICE, COLUMBUS, OHIO, US; 1981, WOLINSKI, JERZY ET AL: "Search for anticholinergic compounds. XX. Synthesis of aminoalkyl o-, m-, and p-hydroxybenzoates and o-, m-, and p-acetoxybenzoates", XP002571760, retrieved from STN Database accession no. 1981:424459 * abstract * -& WOLINSKI, JERZY ET AL: "Search for anticholinergic compounds. XX. Synthesis of aminoalkyl o-, m-, and p-hydroxybenzoates and o-, m-, and p-acetoxybenzoates", ACTA POLONIAE PHARMACEUTICA , 37(3), 275-80 CODEN: APPHAX; ISSN: 0001-6837, 1980, XP008119888, * page 276, table 1: the hydrochloride and the methyliodide of the compound of entry 4 * ----- -/-- | 1 | TECHNICAL FIELDS SEARCHED (IPC) |

The present search report has been drawn up for all claims

1

| Place of search | Date of completion of the search | Examiner |
|---|---|---|
| The Hague | 29 May 2013 | Fitz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 15 5198

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE CA [Online]<br>CHEMICAL ABSTRACTS SERVICE, COLUMBUS, OHIO, US;<br>1966,<br>MACHON, ZDZISLAW ET AL: "Synthesis of benzoylcholine derivatives",<br>XP002571761,<br>retrieved from STN<br>Database accession no. 1966:412012<br>* abstract *<br>& MACHON, ZDZISLAW ET AL: "Synthesis of benzoylcholine derivatives",<br>DISSERTATIONES PHARMACEUTICAE , 17(4), 491-6 CODEN: DIPHAH; ISSN: 0301-1615, 1965,<br>----- | 1 | |
| A | US 6 368 618 B1 (JUN H WON [US] ET AL)<br>9 April 2002 (2002-04-09)<br>* abstract *<br>* claim 9 *<br>----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 29 May 2013 | Fitz, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 15 5198

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2013

| Patent document cited in search report | Publication date | Patent family member(s) | Publication date |
|---|---|---|---|
| EP 0152379 A2 | 21-08-1985 | AU 588798 B2 | 28-09-1989 |
| | | AU 3875385 A | 22-08-1985 |
| | | CA 1246446 A1 | 13-12-1988 |
| | | DK 68585 A | 16-08-1985 |
| | | EP 0152379 A2 | 21-08-1985 |
| | | ES 8607906 A1 | 16-11-1986 |
| | | JP S60190710 A | 28-09-1985 |
| | | ZA 8501111 A | 25-09-1985 |
| GB 958186 A | 21-05-1964 | CH 399486 A | 30-09-1965 |
| | | DE 1179556 B | 15-10-1964 |
| | | GB 958186 A | 21-05-1964 |
| | | LU 42765 A1 | 26-01-1963 |
| US 6368618 B1 | 09-04-2002 | AU 4209400 A | 22-01-2001 |
| | | US 6368618 B1 | 09-04-2002 |
| | | WO 0102015 A1 | 11-01-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6773724 B, Franckowiak
- US 4244948 A, Boghosian
- US 5760261 A, Guttag
- US 4743704 A, Nicolini
- US 5861170 A, Kissel
- US 4012508 A, Burton
- US 4640689 A, Sibalis
- US 5570559 A, Bianco, Sebastiano


### Non-patent literature cited in the description


- **C. H. HENNEKENS et al.** *N. Engl. J. Med.,* 1989, vol. 321, 129
- **T.A.GOSSEL.** *U.S. Pharmacist,* February 1988, 34
- **M.J.THUN et al.** *N. Engl. J. Med.,* 1991, vol. 325, 1393
- PDR Generics. Medical Economics. 1996, 243
- **SUSAN MILOSOVICH et al.** *J. Pharm. Sci.,* 1993, vol. 82, 227
- PDR Generics. Medical Economics. 1996, 242
- **D'AMOUR-SMITH.** *J. Pharmacol. Exp. Ther.,* 1941, vol. 72, 74